# EUROPEAN PATENT APPLICATION

(11) **EP 4 725 385 A1**
(43) Date of publication of application: **15.04.2026**
(21) Application number: 24206152.1
(22) Date of filing: 11.10.2024
(51) Int. Cl.: A61B 1/00, A61B 1/04, G06T 5/50, H04N 23/45, H04N 23/73, H04N 25/534

(54) **METHOD FOR OPTICAL IMAGING, MULTI-SENSOR CAMERA SYSTEM FOR OPTICAL IMAGING AND COMPUTER PROGRAM PRODUCT**

(71) Applicant: Quest Photonic Devices B.V., 1771 SR Wieringerwerf (NL)
(72) Inventor: SOEBRATA, Ferran, 1616 XA Hoogkarspel (NL); KOOPMAN, Thomas, 1013 WR Amsterdam (NL); TIEBIE, Kasper, 1761 AJ Anna Paulowna (NL); BEUKEMA, Glenn, 1705 DD Heerhugowaard (NL)
(74) Representative: Seemann & Partner Patentanwälte mbB

(57) **Abstract**

The invention relates, inter alia, to a method for optical imaging, in particular of a surgical procedure, wherein images are acquired by a multi-sensor camera (40) with a first sensor (A) and a second sensor (B), wherein the first sensor (A) acquires an original image (A1) using a first exposure time (21), wherein the first exposure time (21) is contained in a first measurement interval (22), wherein the second sensor (B) acquires images sequentially, wherein a second exposure time (31) for each image is contained in a second measurement interval (32), wherein the second measurement interval (32) is smaller than the first measurement interval (22), wherein the method further comprises: selecting two images (B2, B3) acquired by the second sensor (B), wherein the two selected images (B2, B3) are a former image (B2) acquired at least partially, in particular fully, during the first measurement interval (22) and a later image (B3) acquired at least partially, in particular fully, after the first measurement interval (22), identifying differences between the two selected images (B2, B3), creating an interpolated image (A1i) by applying the identified differences to the original image (A1) acquired by the first sensor (A), showing the original image (A1) acquired by the first image sensor (A) in an initial part (26) of a first display interval (25) starting after the first measurement interval (22), and showing the interpolated image (A1i) in an end part (27) of the first display interval (25).

## Description

The invention relates to a method for optical imaging, in particular of a surgical procedure, to a multi-sensor camera system for optical imaging, in particular of a surgical procedure, as well as a computer program product.

Imaging has developed into an important and powerful tool, and is in particular used to complement surgical procedures. Optical imaging during surgery allows the medical staff to acquire more information about the patient and its current status as well as to perform precise treatment. For example, in open surgery, optical imaging may provide zoom-ins and magnifications of a surgical area, enhancing a surgeon's vision. In endoscopic or laparoscopic surgery, optical imaging using an endoscope makes it possible to visually inspect the surgical area in the first place.

A significant technique of optical imaging used in the context of medical procedures is fluorescence imaging, in particular fluorescence endoscopy or fluorescence guided surgery. This technique uses specialized dyes, which are excited by at least one excitation light source, to label and highlight certain structures of a surgical area. Typically, excitation wavelengths are in the near infrared, e.g., in a range of 800 nanometers to 1100 nanometers, or in other cases in the visible spectrum, depending on the dye. Surgical staff usually is provided with both an image of the fluorescence emission as well as a standard camera image obtained under white light illumination.

A suitable tool for acquiring simultaneous images of multiple wavebands are multi-sensor cameras. In one embodiment of a multi-sensor camera, a multispectral prism camera, incoming light from a single optical path is split by a prism into two or more separate paths, continuing to propagate towards separate sensors. These sensors are precisely aligned with each other, regardless of motion or a viewing angle of the camera.

Images for surgical procedures are usually recorded continuously as a video, which may be presented as a live image directly to the surgical staff, e.g., on a screen in the operating room. Different sensors of a multi-sensor camera may operate at different frame rates, i.e., acquiring images with differing exposure times. For example, fluorescence emissions are typically weak, so fluorescence images require a longer exposure time in order to obtain sufficient signal, wherein for a white light image a shorter exposure time will usually suffice. Exposure times may also differ depending on the respective wavelength bandwidth.

Such restrictions regarding the exposure times, in particular long exposure times, may slow down a live video shown to surgical staff.

Changes in the field of view or viewing angle as well as instruments moved into the surgical area may only be depicted with a time delay dependent on the respective exposure time. Such delays in the display of the fluorescence images slow down the procedure, thereby prolonging the duration of the surgical procedure and leading to worse patient outcomes. Other events changing the content of acquired images may be, for example, the manipulation of patient's tissue by medical instruments, movement of tissue due to the patient's breathing, moving or muscle contractions as well as bleeding. Time delays in imaging may hinder a quick response addressing such events for the benefit of the patient.

In the context of the aforementioned, it is an object of the invention to improve the quality of optical imaging of a surgical procedure using a multi-sensor camera, in particular to improve the imaging speed.

The object is solved by a method for optical imaging, in particular of a surgical procedure, wherein images are acquired by a multi-sensor camera with a first sensor and a second sensor, wherein the first sensor acquires an original image using a first exposure time, wherein the first exposure time is contained in a first measurement interval, wherein the second sensor acquires images sequentially, wherein a second exposure time for each image is contained in a second measurement interval, wherein the second measurement interval is smaller than the first measurement interval, wherein the method further comprises:
- selecting two images acquired by the second sensor, wherein the two selected images are a former image acquired at least partially, in particular fully, during the first measurement interval and a later image acquired at least partially, in particular fully, after the first measurement interval,
- identifying differences between the two selected images,
- creating an interpolated image by applying the identified differences to the original image acquired by the first sensor,
- showing the original image acquired by the first image sensor in an initial part of a first display interval starting after the first measurement interval and showing the interpolated image in an end part of the first display interval.

In this context, a measurement interval is synonymous with the time stretching from the start of acquiring an image to the time of the start of acquiring a subsequent image. The duration of the measurement interval is therefore the inverse of the frame rate.

The method helps minimize time delays for displaying acquired images.

A basic idea of the invention is to combine both images of two optical sensors with differing exposure times and frame rates in order to create an interpolated image for the slower one of the image sources. The interpolated image is based on an original image of the image sensor with a longer exposure time and lower frame rate, wherein the image is processed using information from the image sensor with a shorter exposure time and higher frame rate. In this context, the relative terms "shorter" and "longer" are to be understood as meaning "shorter" or "longer" relative to each other in the context of the present disclosure, such as in "the shorter (or, respectively, longer) one of the first and the second measurement intervals".

Furthermore, the exposure time of a sensor will typically be a bit less than the measurement interval, since the sensors usually have a duty cycle of less than 100% due to the fact that no light is acquired while the image sensor is read out. Furthermore, varying lighting conditions may influence the exposure time for correct exposure while keeping the frame rate, and thus the measurement intervals, constant.

Between displaying regularly recorded images of a longer exposure time, an interpolated image may be displayed containing features that have evolved after the exposure time of the first regular image. By applying such a frame rate interpolation for two image sensors of differing frame rates, time delays in displaying the information detected by the respective image sensors may be minimized.

Preferably, the multi-sensor camera is a prism-based multi-sensor camera. This allows to map the same region, in particular of a surgical area, by both the first sensor and the second sensor using the same optical path. In particular, the first sensor is a first image sensor and the second sensor is a second image sensor.

A continuous display of acquired images is achieved, if the first display interval follows directly to the first measurement interval. This way, a sequence of a first measurement interval and a first display interval has a duration of two first exposure times. In particular, the initial part of the first display interval has the same or a longer duration than the end part of the first display interval.

The images may be acquired during a surgical procedure, but may also come from other types of image sources, such as multicam streams whose different cameras have different frame rates. In the case of a surgical procedure, the surgical procedure may, in various embodiments, be at least one of open surgery, endoscopic surgery and laparoscopic surgery. For these types of procedures, reducing the time delay through interpolated images is particularly beneficial.

In an embodiment, the multi-sensor camera is a multispectral camera, wherein the first sensor and the second sensor map different wavebands of optical radiation, wherein in particular the first sensor and/or the second sensor detect near-infrared light, in particular for near-infrared fluorescence imaging. Acquiring images of different optical spectra, in particular by using fluorescence imaging, allows to obtain profound live information about the patient with minimized time delay. In particular, the first sensor and/or the second sensor is configured to only detect near-infrared light. In particular, at least one of the sensors may be configured to detect at least one of ultraviolet light, in particular in a range of 10 to 400 nanometers, visible light, in particular in a range of 400 to 780 nanometers, and infrared light, in particular in a range of 750 to 1400 nanometers.

In particular, the second sensor acquires images in a waveband of white light. Typically, white light imaging allows for high frame rates and low exposure times, such that it allows to provide information for an imaging technique with lower frame rates, such as fluorescence imaging.

A video stream with minimized time delay may be provided, if the method steps are performed repeatedly, in particular for each original image acquired by the first sensor. This way, each image acquired by the first sensor is accompanied by an interpolated image shown in a later part of the first display interval. In another embodiment, the method steps may be performed repeatedly for every other original image acquired by the first sensor.

In embodiments, the first measurement interval is an integer multiple of the second measurement interval or a non-integer multiple of the second measurement interval. If the measurement intervals have an integer ratio, e.g., 2:1 or 3:1, the second sensor will acquire two, three or more images in the time the first sensor acquires one image, and the first of the two, three or more images acquired by the second sensor may be timed to start at the same time as the acquisition of the image by the first sensor. Therefore, for each of the original images acquired by the first sensor an interpolated image may be obtained in a similar manner, selecting two images of the second sensor with the same respective time interval with respect to the respective start of the respective first measurement interval. If, on the other hand, the measurement intervals have a non-integer ratio, e.g., 3:2 or 5:2, the start of image acquisition by the first and second sensors will be synchronized only every other time the first sensors start acquiring an image. Therefore, in such a setting the selected images of the second sensor may have to be determined individually.

Energy-efficiency and speed of the method are further enhanced, if identifying differences between the two selected images and/or creating the interpolated image by applying the identified differences to the original image is supported by a hardware acceleration unit, in particular a hardware video acceleration unit comprised by the multi-sensor camera. Hardware acceleration provides faster program execution by using specialized hardware, in particular video hardware. The faster the optical difference between the two selected images of the second sensor is identified and the faster the optical difference is applied to the original image of the first sensor, the faster the interpolated image may be displayed to the user, keeping time lag in the display low.

The images are processed efficiently and timely, if identifying differences between the two selected images and/or creating the interpolated image by applying the identified differences to the original image comprises applying video compression techniques, in particular inter frame prediction techniques, to the two selected images and/or the original image.

In video compression, it is a known technique to define key frames, for which all information about all pixels of the corresponding image is stored in the video file. For the subsequent frames, in other words images, only differences with regard to the key frame are stored. This way, for similar frames, e.g., showing the same scene of a film, a lot of storage capacity may be saved. In this context, many techniques have been developed to identify differences between images, e.g., inter frame prediction techniques such as a block matching algorithm. Typically, such methods are comprised in video compression standards. Applicable video compression standards are in particular, but not limited to, H.264, H.265, VP9, AV1 and MPEG-2 Video. In an embodiment, identifying differences between the two selected images may comprise auto-correlating the two selected images.

For identifying differences between the two selected images, techniques of video compression suitable for compression are applied. Starting from two complete images, the differences are calculated, as if a video is compressed with the first of the two selected images being a key frame. For creating the interpolated image, techniques of video compression suitable for decompression are applied. Starting from the original image, the optical differences are applied in a way similar to the playback of a compressed video file with the original image being a key frame and the optical differences being the information stored about a subsequent frame.

Optical imaging and the usability for the users is further enhanced, if a frame rate of the first sensor is at least 5 images per second, in particular 10 frames per second, in particular 20 frames per second, in particular 30 frames per second, in particular 60 frames per second and/or wherein a frame rate of the second sensor is at least 5 images per second, in particular 10 frames per second, in particular 20 frames per second, in particular 30 frames per second, in particular 60 frames per second, in particular 90 frames per second, in particular 120 frames per second. Frame rates of 5 to 120 frames per second correspond to maximum exposure times of 200 milliseconds to 8.33 milliseconds.

The object is also solved by a multi-sensor camera system for optical imaging, in particular of a surgical procedure, comprising a multi-sensor camera with a first sensor and a second sensor, wherein the multi-sensor camera system is configured to carry out the method as described above. The multi-sensor camera system provides the same technical effects and technical advantages as the method for optical imaging.

In particular, the multi-sensor camera system comprises an endoscope and/or a laparoscope.

Furthermore, the object is also solved by a computer program product comprising instructions which, when the program is executed by a computer, in particular of the above described multi-sensor camera system for optical imaging, cause the computer to carry out the method for optical imaging as described above. The computer program product provides the same technical effects and technical advantages as the method for optical imaging.

Finally, the object is also solved by a surgical method, in particular for open surgery, for endoscopic surgery and/or for laparoscopic surgery, applying the method for optical imaging as described above for imaging of the surgical procedure.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
Fig. 1 a schematic representation of synchronous image rendering with image sensors with different exposure times according to the state of art,
Fig. 2 a schematic representation of asynchronous image rendering with image sensors with different exposure times according to the state of art,
Fig. 3 a schematic representation of a first embodiment of a method for optical imaging of a surgical procedure,
Fig. 4 a schematic representation of a second embodiment of a method for optical imaging of a surgical procedure, and
Fig. 5 a schematic representation of a first embodiment of a multi-sensor camera system.

In the drawings, the same or similar types of elements or respectively corresponding parts are provided with the same reference numbers in order to prevent the item from needing to be reintroduced.

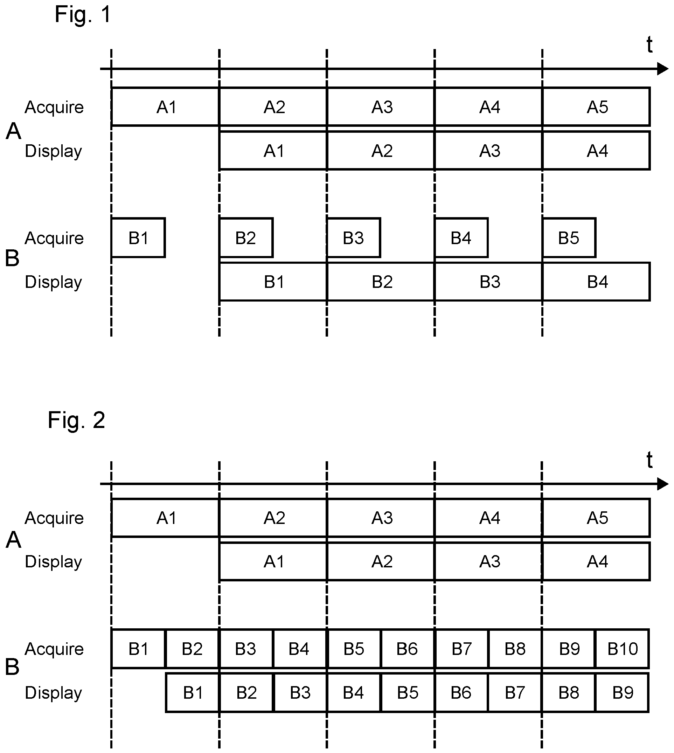

Figure 1 depicts a schematic representation of synchronous image rendering with image sensors with different exposure times according to the state of art. A multi-sensor camera comprises two sensors, a first sensor A and a second sensor B. Both sensors A, B acquire images, which are subsequently displayed to a user. The diagram shown in Fig. 1 shows a timeline progressing in time t, as indicated by the arrow on the top. The dashed lines running vertically act a guide to the eye, delineating subsequent frames.

For acquiring images, the first sensor A has a measurement interval twice that of the second sensor B. For example, sensor B might be a sensor tasked with white light imaging, whereas sensor A is tasked with fluorescence imaging, which due to its low intensity needs longer exposure times.

In this case, whereas the first sensor A acquires images A1, A2, A3, ... continuously, the second sensor B is always prompted to start acquiring images B1, B2, B3, ... at the same times as first sensor A, thus ensuring synchronicity of the images A1, A2, A3, ... and B1, B2, B3, .... This means, however, that due to the shorter exposure time, or respectively, measurement interval, the second sensor B is in stand-by during each subsequent frame for a period of time determined by the difference of frame rates between the first sensor A and the second sensor B before capturing the next image.

The images acquired by both sensors A, B are displayed subsequently to their capture. When both sensors A, B start acquiring a new image, the previously captured images are displayed simultaneously. Despite the fact that the second sensor B could acquire and display more images, it is synchronized to the first sensor A. Therefore, this way of rendering images is called synchronous rendering.

Figure 2 shows a schematic representation of asynchronous image rendering with image sensors with different frame rates according to the state of art. Fig. 2 is set up in a similar fashion as Fig. 1. For a first sensor A and a second sensor B of a multi-sensor camera, a timeline is shown for acquiring and displaying images, with time advancing to the right in the paper-plane.

The technical specifications of both sensors A, B are similar to those shown in Fig. 1. The first sensor A operates with a measurement interval that is twice the measurement interval of second sensor B, or, respectively, half the frame rate. In contrast to Fig. 1, both sensors A, B continuously acquire images and display the acquired images as soon as available. Second sensor B does not wait for periods of time in a standby mode, but instead operates at a frame rate double the first sensor A's frame rate.

As consequence, images displayed to the user are not shown at the same time. Instead, images of second sensor B are refreshed twice while images of first sensor A are updated only once. Therefore, both displays do not depict the same points in time, e.g., when B3 is shown to the user, sensor A shows A1, which finished acquisition before capturing B3 started. This kind of rendering the acquired images is called asynchronous image rendering.

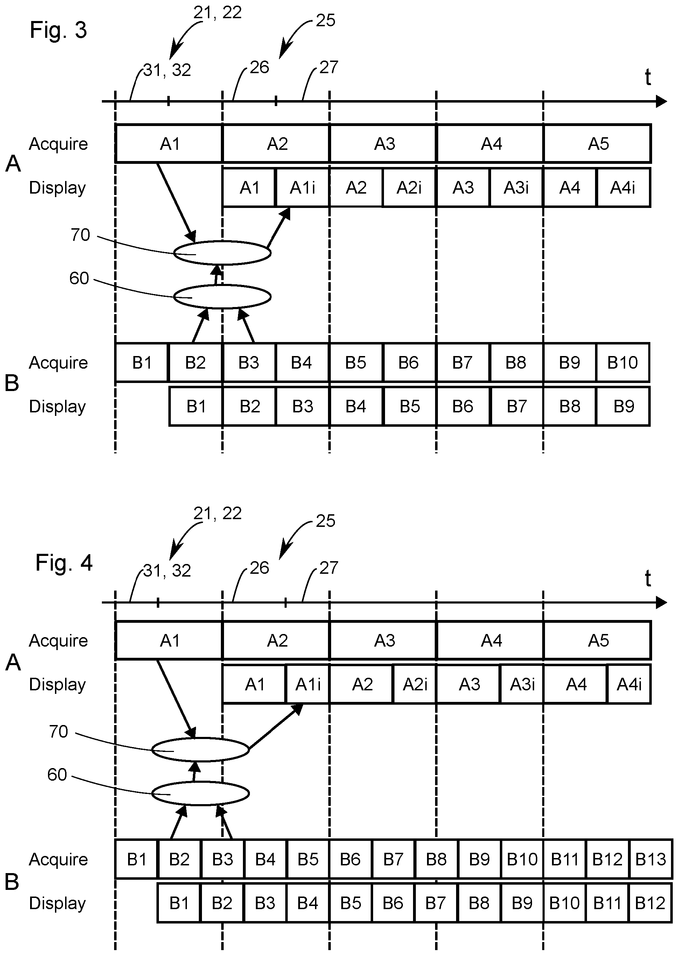

Figure 3 depicts a schematic representation of a first embodiment of a method for optical imaging of a surgical procedure. A multi-sensor camera is equipped with a first sensor A and a second sensor B. For example, the first sensor A may be a sensor sensitive to near-infrared radiation for fluorescence imaging and the second sensor B may be a sensor sensitive to white light for traditional optical imaging of visible light.

The first sensor A acquires images A1, A2, A3, ... sequentially with a first exposure time 21, which, in this case, defines a first measurement interval 22. This is for illustrative purposes and assumes a duty cycle of 100%. In reality, the exposure time will typically be shorter than the measurement interval, with duty cycles, meaning ratios of exposure time to length of measurement intervals, being less than 100%.

In this example, the first exposure time 21 is twice as long as second sensor B's second exposure time 31. For example, first sensor A may operate at 30 frames per second and second sensor B may operate at 60 frames per second. As the ratio of both exposure times, or, resp., measurement intervals, is 2:1, the first exposure time 21 or measurement interval 22 is an integer multiple of the second exposure time 31 or measurement interval 32.

In Fig. 3, for the sake of readability, only the first occurrences in time of the exposure times 21, 31, the measurement intervals 22, 32 as well as the display intervals 25 are labelled with references. As the method may operate for each original image A1, A2, A3, ..., these time intervals could be labelled for each of the images acquired.

According to the state of art (c.f. Fig. 2), image A1 would be displayed during the full time interval during which image A2 is captured by first sensor A. However, here the original image A1 is displayed in an initial part 26 of the first display interval 25 and an interpolated image A1i is displayed in the end part 27 of the first display interval 25.

For creating the interpolated image A1i, two images B2, B3 acquired by the second sensor B are selected and processed. The former selected image B2 is fully acquired during the first measurement interval 22, i.e., during the exposure time of the original image A1. The later selected image B3 is fully acquired after the first measurement interval 22, i.e., only during the exposure time of the image A2 acquired after the original image A1. This way, changes after the acquisition of the original image A1 may be included in the interpolated image A1i. Changes in the images, for example changes in the viewing angle or alterations in the patient's tissue caused by treatment, are thus displayed to the user more quickly.

Having selected the two images B2, B3, the next step is to identify (reference 60) differences between the two selected images B2, B3. Such identification of differences may be performed using standard techniques of video compression, such as inter frame prediction techniques. In this embodiment, the compression techniques of the video standard H.264 are implemented.

This result comprising the differences between the two selected images B2, B3 is applied (reference 70) to the original image A1. From the combination of the original image A1 and the identified differences an interpolated image A1i is generated. In this embodiment, the decompression techniques of the video standard H.264 are implemented to apply the identified differences to the original image A1.

During the first display interval 25, both the original image A1 and the interpolated image A1i are displayed subsequently. In an initial part 26 of the first display interval 25 the original image A1 is displayed. While showing this image, image B3 is acquired and the interpolated image A1i is computed. Subsequently, in an end part 27 of the first display interval 25, the interpolated image A1i is displayed, in parallel with image B3.

In a variation to the sequence shown in Fig. 3, the display of the image sequence B1, B2, B3, ..., may also be delayed by a single B-frame, such that frame B1 is displayed at the same time as A1, B2 along with A1i, and so forth. Although this measure will add a slight delay in the display, it provides enhanced synchronicity between the two video feeds. This is because frames B2 and B3 have been used to identify "before/after" image changes that are applied to image A1 to produce A1i. The "before/after" pairs B2/B3 and A1/A1i are displayed at the same time, meaning that any changes in the images show up in both streams at the same time.

Fig. 4 depicts a schematic representation of a second embodiment of a method for optical imaging of a surgical procedure. In general, Fig. 4 is set up in a similar fashion as Fig. 3.

The ratio of exposure times for the embodiment shown in Fig. 4 is 5:2. Hence, the first exposure time 21 is a non-integer multiple (5:2 = 2.5) of the second exposure time 31. For example, the first exposure time 21 may be 100 milliseconds, corresponding to 10 frames per second assuming there is no dead time in the sequence, and the second exposure time 31 may be 40 milliseconds, corresponding to 25 frames per second under the same assumption.

Similar to the embodiment shown in Fig. 3, an interpolated image A1i is generated by first selecting images B2 and B3. Here, former image B2 is fully acquired during the first measurement interval 22 and the later image B3 is partially acquired during the first measurement interval 22. Both selected images B2, B3 are compared (reference 60) and their identified differences are applied (reference 70) to the original image A1 in order to create the interpolated image A1i.

The interpolated image A1i is displayed in an end part 27 of the first display interval 25, wherein the end part 27 is shorter than the initial part 26 of the first display interval 25.

For creating the interpolated image A2i, images B5 and B6 are selected. As a consequence of the non-integer ratio of the frame rates, in contrast to image B3, image B6 is fully acquired after the measurement interval of the image A2.

For the interpolated image A3i, a similar selection as for A1 can be made by selecting images B7 and B8. As the ratio of exposure times is 5:2, after acquiring two images, the situation is similar to the beginning, wherein both sensors A, B start acquiring images at the same time.

As explained before, in all the examples, it is assumed that the sensors have a duty cycle of 100%, i.e., the exposure time is the exact invers of the frame rate. In reality, the duty cycle may be smaller than 100% due to the time necessary to read out the image date, or due to varying amounts of lighting, leading to a decrease in exposure time while keeping the frame rate constant.

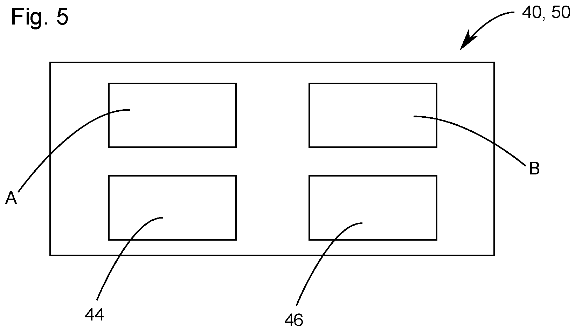

Fig. 5 depicts a schematic representation of a first embodiment of a multi-sensor camera system 50 for optical imaging of a surgical procedure. The multi-sensor camera system 50 comprises a multi-sensor camera 40. The multi-sensor camera 40 comprises a first sensor A and a second sensor B, wherein first sensor A is configured to detect ultra-violet light and second sensor B may be configured to detect an optical wavelength band of green color. Furthermore, the multi-sensor camera 40 comprises a hardware acceleration unit 44, which is configured to process H.265 video data. The hardware acceleration unit 44 is used to apply the identified differences between selected images B2, B3 and to apply the differences to an original image A1 in order to generate an interpolated image A1i. In order to process the method for optical imaging, including operating the hardware acceleration unit 44, the multi-sensor camera system 50 comprises a processing unit 46.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 21: first exposure time
- 22: first measurement interval
- 25: first display interval
- 26: initial part of the first display interval
- 27: end part of the first display interval
- 31: second exposure time
- 32: second measurement interval
- 40: multi-sensor camera
- 41: first sensor
- 42: second sensor
- 44: hardware acceleration unit
- 46: processing unit
- 50: multi-sensor camera system
- 60: identify differences
- 70: apply differences
- A: first sensor
- B: second sensor
- A1: original image
- A1i: interpolated image
- B2: former selected image
- B3: later selected image
- t: time

## Claims

1. Method for optical imaging, in particular of a surgical procedure, wherein images are acquired by a multi-sensor camera (40) with a first sensor (A) and a second sensor (B),
wherein the first sensor (A) acquires an original image (A1) using a first exposure time (21), wherein the first exposure time (21) is contained in a first measurement interval (22), wherein the second sensor (B) acquires images sequentially, wherein a second exposure time (31) for each image is contained in a second measurement interval (32), wherein the second measurement interval (32) is smaller than the first measurement interval (22), wherein the method further comprises:
- selecting two images (B2, B3) acquired by the second sensor (B), wherein the two selected images (B2, B3) are a former image (B2) acquired at least partially, in particular fully, during the first measurement interval (22) and a later image (B3) acquired at least partially, in particular fully, after the first measurement interval (22),
- identifying differences between the two selected images (B2, B3),
- creating an interpolated image (A1i) by applying the identified differences to the original image (A1) acquired by the first sensor (A),
- showing the original image (A1) acquired by the first image sensor (A) in an initial part (26) of a first display interval (25) starting after the first measurement interval (22) and showing the interpolated image (A1i) in an end part (27) of the first display interval (25).

2. Method according to claim 1, wherein the surgical procedure is at least one of open surgery, endoscopic surgery and laparoscopic surgery.

3. Method according to claim 1 or 2, wherein the multi-sensor camera (40) is a multispectral camera, wherein the first sensor (A) and the second sensor (B) map different wavebands of optical light, wherein in particular the first sensor (A) and/or the second sensor (B) detect near-infrared light, in particular for near-infrared fluorescence imaging.

4. Method according to one of the claims 1 to 3, wherein the method steps are performed repeatedly, in particular for each original image (A1, A2, A3, ...) acquired by the first sensor (A).

5. Method according to one of the claims 1 to 4, wherein the first measurement interval (22) is an integer multiple of the second measurement interval (32) or a non-integer multiple of the second measurement interval (32).

6. Method according to one of the claims 1 to 5, wherein identifying differences between the two selected images (B2, B3) and/or creating the interpolated image (A1i) by applying the identified differences to the original image (A1) is supported by a hardware acceleration unit (44), in particular a hardware video acceleration unit comprised by the multi-sensor camera (40).

7. Method according to one of the claims 1 to 6, wherein identifying differences between the two selected images (B2, B3) and/or creating the interpolated image (A1i) by applying the identified differences to the original image (A1) comprises applying video compression techniques, in particular inter frame prediction techniques, to the two selected images (B2, B3) and/or the original image (A1).

8. Method according to one of the claims 1 to 7, wherein a frame rate of the first sensor (A) is at least 5 images per second, in particular 10 frames per second, in particular 20 frames per second, in particular 30 frames per second, in particular 60 frames per second and/or wherein a frame rate of the second sensor (B) is at least 5 images per second, in particular 10 frames per second, in particular 20 frames per second, in particular 30 frames per second, in particular 60 frames per second, in particular 90 frames per second, in particular 120 frames per second.

9. Multi-sensor camera system (50) for optical imaging, in particular of a surgical procedure, comprising a multi-sensor camera (40) with a first sensor (A) and a second sensor (B), wherein the multi-sensor camera system (40) is configured to carry out the method of claims 1 to 8.

10. Multi-sensor camera system (50) according to claim 9, comprising an endoscope and/or a laparoscope.

11. Computer program product comprising instructions which, when the program is executed by a computer, in particular a computer of a multi-sensor camera system according to claim 9 or 10, cause the computer to carry out the method of claims 1 to 8.
